# EUROPÄISCHE PATENTANMELDUNG

(11) **EP 2 215 994 A1**
(43) Veröffentlichungstag der Anmeldung: **11.08.2010**
(21) Anmeldenummer: 10000797.0
(22) Anmeldetag: 27.01.2010
(51) Int. Cl.: A61F 5/01, A61H 1/00, A61H 23/00

(54) **Orthese**

(30) Priorität: 05.02.2009 DE 102009007710
(71) Anmelder: Pohlig GmbH, 83278 Traunstein (DE)
(72) Erfinder: Pohlig, Kurt, 83278 Traunstein (DE)
(74) Vertreter: Paustian, Othmar

(57) **Zusammenfassung**

Die Erfindung betrifft eine Orthese mit mindestens einem Vibrationselement (6, 11, 21) zur Erzeugung von Vibrationen in zumindest einem Abschnitt der Orthese. Dieser Abschnitt ist dazu eingerichtet, im an einen Körperteil angelegten Zustand der Orthese, auf Kontakt anzuliegen.

## Beschreibung

Die Erfindung betrifft eine Orthese.

Orthesen sind allgemein bekannt. Sie dienen in der Regel zur Stützung von Körperteilen bzw. zur Korrektur von Gelenkstellungen. Sie werden insbesondere zur Stützung von Extremitäten und Gelenken und nach Operationen eingesetzt.

Bekannt sind insbesondere Unterschenkelorthesen. Diese weisen üblicherweise ein oder mehrere Schalenteile zum Umgreifen des Unterschenkels und ein Fußteil zur Aufnahme des Fußes auf. Die stützenden bzw. umgreifenden Bestandteile einer Orthese verfügen, damit sie ihre Funktion erfüllen können, über eine gewisse Steifigkeit bzw. Rigidität. Ein typisches Material für Schalenteile sind Faserverbundwerksoffe, insbesondere karbonfaserverstärkte Laminate.

Die einzelnen Schalenteile werden an den Körper angelegt und etwa über Riemen oder Klettverschlüsse fixiert.

Die Zeit, die die Orthese getragen werden sollte, also die Dauer der Behandlung, der Erfolg derselben und auch das Wohlbefinden der behandelten Person hängen bisher vor Allem von der - gegebenenfalls postoperativen - Ausgangssituation und der Konstitution dieser Person ab.

Der Erfindung liegt die Aufgabe zugrunde, eine Orthese anzugeben, die die Dauer der Behandlung verkürzen, den Erfolg derselben verbessern oder sich positiv auf das Wohlbefinden der zu behandelnden Person auswirken kann.

Die Aufgabe wird gelöst durch eine Orthese mit mindestens einem Vibrationselement zur Erzeugung von Vibrationen in zumindest einem Abschnitt der Orthese, wobei dieser Abschnitt dazu eingerichtet ist, im an einen Körperteil angelegten Zustand der Orthese auf Kontakt anzuliegen.

Bevorzugte Ausgestaltung der Erfindung sind in abhängigen Ansprüchen angegeben und werden im Folgenden ebenfalls näher erläutert:

Die Erfindung beruht auf der Idee, eine Orthese mit einem Vibrationselement zu versehen.

Vibrationen können sich positiv auf den Körper, insbesondere auf dessen Weichteile, auswirken. Von Vibrationen wird es gemeinhin erwartet, dass sie einzelne Muskeln oder auch ganze Muskelgruppen entspannen und gegebenenfalls sogar stärken, das neuronale System stärken und die Durchblutung fördern können. Vibrationen wird auch die Förderung von Wundheilungsprozessen zugeschrieben.

Eine erfindungsgemäße Orthese erlaubt die Behandlung eines Orthesenträgers mit Vibrationen, was die genannten Vorteile mit sich bringt. Diese Vorteile sind gerade für Orthesenträger von besonderer Relevanz, z.B. wenn Orthesen nach Operationen getragen werden. Gerade postoperativ ist jede Verbesserung der Behandlung, insbesondere die Förderung des Wundheilungsprozesses bei angelegter Orthese, von besonderer Bedeutung, da die postoperative Phase schon ohne Orthese für die zu behandelnde Person voller Unannehmlichkeiten ist und es auch nicht selbstverständlich ist, dass die Wunden gut verheilen

Je nach Art und Ort des Anbringens des Vibrationselements kann gegebenenfalls sogar gezielt lokal auf den Körper eingewirkt werden. Beispielsweise wird ein Vibrationselement, das an der Sohle eines Fußteils einer Unterschenkelorthese angebracht ist, sich vor allem auf die Unterseite des Fußes auswirken. Da die vergleichsweise steifen Materialien, aus denen Orthesen typischerweise gebildet sind, Schwingungen recht gut weiterleiten, wirken sich die Vibrationen aber auch noch in einem gewissen Abstand von dem Vibrationselement aus.

Es ist zu erwarten, dass die erfindungemäße Orthese sich nicht nur vorteilhaft für die Behandlung von orthopädischen bzw. neuroorthopädischen Erkrankungen einsetzen lässt, sondern sich auch positiv auf Durchblutungsstörungen, etwa im Zusammenhang mit Diabetes mellitus, auswirkt.

Bei dem Vibrationselement kann es sich etwa um einen vergleichsweise kleinen hohlen Zylinder handeln, in dessen Inneren sich ein Antrieb für eine Unwucht befindet. Bei der Unwucht kann es sich etwa um einen Exzenter oder um ein hin- und herschwingendes Gewicht handeln. Das Vibrationselement kann etwa auf der Außenseite der Orthese befestigt sein oder auch teilweise in dieser versenkt sein. Besonders geeignet ist eine Befestigung, die von Außen durch eine Schale der Orthese hindurchgreift und das Vibrationselement an der Innenseite der Orthese verankert. Auch damit der Orthesenträger durch die Verankerung nicht gestört wird, bietet es sich an, die Orthese mit einer weichen Innenhülle ("Liner") auszustatten. Es ist auch denkbar, das Vibrationselement über eine Halterung an der Außenseite der Orthese zu befestigen.

Zur Übertragung der Vibrationen von der Orthese auf den Körper ist ein schwingungsübertragender Kontakt zwischen der Orthese und dem Körperteil erforderlich, auf das mit den Vibrationen eingewirkt werden soll. Der Begriff "Kontakt" steht also für eine schwingungsübertragungsfähige Anlage der Orthese an den entsprechenden Körperteil. Für einen solchen schwingungsübertragungsfähigen Kontakt ist es nicht erforderlich, dass die Orthese auf nackter Haut aufliegt. Es können sich beispielsweise Lagen aus Textil zwischen der Orthese und dem Körper des Orthesenträgers befinden. Der Begriff "Kontakt" kann hier entsprechend als "schwingungsübertragungsfähige Verbindung bzw. Anlage" gelesen werden.

Vorzugsweise weist die Orthese eine Fixiereinrichtung zur Sicherung des Kontakts des vibrierenden Abschnitts mit dem Körper auf.

Diese bevorzugte Ausgestaltung beruht auf der Einsicht, dass der Kontakt zwischen der Orthese und dem Körper unter Umständen teilweise verloren gehen kann. Wo der Kontakt dann nicht gegeben ist, können die Vibrationen nicht unmittelbar auf den Körper einwirken. Eine möglichst großflächige und zuverlässige Anlage im Kontakt mit dem Körper ist günstig, um eine gute Übertragung der Vibrationen von der Orthese auf den zu behandelnden Körperteil zu gewährleisten.

Es ergibt sich beispielsweise bei einer herkömmlichen Unterschenkelorthese mit einem herkömmlichen Fußteil, dass sich die Sohle des Fußes von der Unterseite des Fußteils ablöst, wenn sich die zu behandelnde Person in liegender Position befindet. Dabei hebt sich die Ferse etwas an, und es verbleibt lediglich ein Kontakt zwischen der vorderen Fußregion, oder ggf. sogar nur den Zehen, und dem Fußteil.

Dieses Phänomen, also eine Verringerung der Kontaktfläche zwischen Körper und Orthese in bestimmten Lagen, ist allgemeiner Natur und nicht auf die obige zur Veranschaulichung herangezogene Situation beschränkt.

Da Orthesen oft postoperativ eingesetzt werden und die operierten Personen nach der Operation oft einige Zeit liegend verbringen, ist es besonders vorteilhaft, dass die Orthese auch im Liegen hinreichend großflächig an dem zu behandelnden Körperteil anliegt, um mit den Vibrationen gut auf diesen Körperteil einwirken zu können.

Bei einer weiteren bevorzugten Ausführungsform weist die Fixiereinrichtung eine zumindest abschnittweise ringförmige Fassung zum zumindest teilweisen Umgreifen einer Extremität auf. Diese Ausführungsform beruht auf der Idee, den Kontakt der Orthese an den zu behandelnden Körperteil mit Hilfe einer zumindest abschnittweise ringförmigen Fassung zum Umgreifen einer Extremität, etwa des Fußes, zu gewährleisten. Die Fassung unterbindet durch das zumindest teilweise Umgreifen ein Ablösen der Orthese von dem zu behandelnden Körperteil; dies gilt insbesondere für den Fall, dass der zu behandelnde Körperteil die umgriffene Extremität ist. Auch einem Verrutschen der Orthese insgesamt wird so entgegengewirkt. Daher wirkt die zumindest abschnittweise ringförmige Fassung auch einer durch die Vibration der Orthese verursachten Bewegung derselben entgegen. Extremitäten eignen sich aufgrund ihres im Vergleich zum Rumpf geringeren Durchmessers gut dazu, von einer Fassung umgriffen zu werden.

Die zumindest abschnittweise ringförmige Fassung ist so ausgelegt, dass sie den Kontakt der Orthese im Bereich dieser Fassung an die umgriffene Extremität sichert. In der Regel bewirkt dies auch eine Verbesserung der Anlage der Orthese im Ganzen. Dazu umgreift die zumindest abschnittweise ringförmige Fassung vorzugsweise zumindest zwei Drittel des Umfangs einer Extremität, besser noch vier Fünftel derselben.

Unter einem "Ring" im Sinne der Erfindung sind sämtliche Formen zu verstehen, die die betreffende Extremität zirkulär umgreifen, also auch der Form der jeweiligen Extremität angepasste Tuben beziehungsweise Hülsen. Wie bereits erwähnt, ist es für den Erfolg dieser bevorzugten Ausgestaltung nicht zwingend erforderlich, dass der Ring vollständig um die Extremität geschlossen ist; es kann beispielsweise ein Spalt verbleiben.

Es ist besonders bevorzugt, wenn die Fassung einen geschlossenen Ring zum Umgreifen der Extremität aufweist. Die geschlossene Form bewirkt eine besonders gute, also großflächige und stabile, Anlage der Orthese. In der Orthopädietechnik wird auch von einer "zirkulären" Fassung gesprochen.

Orthesen werden insbesondere häufig nach Operationen im Sprunggelenksbereich eingesetzt. Eine entsprechende Orthese weist typischerweise eine Unterschenkelschale bzw. Unterschenkelhülse und ein Fußteil auf. Die Patienten werden nach einer Sprunggelenksoperation zumindest zeitweise liegend gelagert. Hier ergibt sich die bereits oben geschilderte Situation, dass sich die Sohle des Fußes bei einem herkömmlichen Fußteil von diesem ablösen kann, da die Schwerkraft den Patienten im Liegen nicht ins Fußbett drückt.

Daher ist bei einer besonders bevorzugten Ausführungsform die Fixiereinrichtung eine Fußfassung zum Umgreifen eines Fußes, entweder zumindest abschnittweise ringförmig oder mit einem geschlossenen Ring.

Ein Verrutschen der Orthese, insbesondere in axialer Richtung (nach oben oder unten entlang der Beinachse) kann mit der, insbesondere geschlossenen, ringförmigen Fußfassung besonders zuverlässig abgeschwächt bzw. sogar vollständig unterbunden werden.

Allgemein eignen sich die Ausführungsformen mit ringförmiger Fußfassung gut zur Stabilisierung und Fixierung der unteren Extremität. Dies ist nicht nur postoperativ bei Sprunggelenksverletzungen von Relevanz, sondern insbesondere auch, wenn intraartikuläre Fehlstellungen korrigierend zu beeinflussen und/oder die Extremität auch aus anderen Gründen optimal zu stützen ist, beispielsweise im Anschluss an eine Gliedmaßenverlängerung, nach Abnahme eines Fixateur extern.

Insbesondere auch wegen der körpernahen Lage der ringförmigen Fußfassung können auch unerwünschte Bewegungen des Beines in der Orthese abgeschwächt oder auch vollständig unterdrückt werden. Dies betrifft etwa Bewegungen des Beines relativ zu dem Fußteil, der Unterschenkelhülse oder aber auch der Oberschenkelhülse, soweit vorhanden.

Insgesamt ermöglichen es die Ausgestaltungen der Erfindung mit Fixiereinrichtung, besonders wirksam und zuverlässig mit Vibrationen auf den Körper des Orthesenträgers einzuwirken.

Menschen reagieren unterschiedlich auf Vibrationen. Das betrifft sowohl die rein therapeutischen Wirkungen der Vibrationen als auch das Wohlbefinden der Person. Daher ist das Vibrationselement vorzugsweise so ausgelegt, dass die Schwingungsfrequenz und die Schwingungsamplitude bzw. Intensität des Vibrationselements vorgebbar ist. Dies kann etwa durch eine Einstellmöglichkeit an dem Vibrationselement realisiert werden. Idealerweise sind jedoch die Zeitverläufe der Schwingungsfrequenz und/oder Amplitude des Vibrationselements, gegebenenfalls auch in Kombination mit der eben genannten Einstellmöglichkeit, im Vorfeld programmierbar. Die Zeitverläufe der Eigenschaften der Vibration können etwa zuvor auf einem Rechner gestaltet werden und dann drahtlos oder über ein Datenübertragungskabel auf das Vibrationselement übertragen werden.

Es ist auch bevorzugt, mehrere Vibrationselemente an einer Orthese zu verwenden. So können unterschiedliche Bereiche gezielt gleichzeitig mit Vibrationen behandelt werden.

Vorteilhaft kann auch eine Schwingungsentkopplung zwischen Teilen der Orthese sein, etwa wenn ein Teil der Orthese besonders intensiv in Schwingungen versetzt werden soll und ein anderer Teil weniger oder gar nicht. Die Teile der Orthese können dazu mit Schwingungsdämpfern entkoppelt werden. Beispielsweise kann bei einer Unterschenkelorthese mit Fußteil dasselbe durch einen Gummipuffer von der Unterschenkelhülse entkoppelt werden. Eine Entkopplung von Teilen einer Schale in sich lässt sich über eine dämpfende Verbindung der Teile, etwa aus Gummi, bewerkstelligen.

Die in der vorangehenden und in der folgenden Beschreibung offenbarten Einzelmerkmale können auch in anderen als den gezeigten Kombinationen erfindungswesentlich sein.

Im Folgenden soll die Erfindung auch anhand von Ausführungsbeispielen näher erläutert werden, ohne dabei die Erfindung auf diese Beispiele einschränken zu wollen.
Figur 1 zeigt den unteren Teil einer Unterschenkelorthese mit ringförmigem Fußteil mit einem Vibrationselement.
Figur 2 zeigt in Aufsicht eine ringförmige Fußfassung einer Unterschenkelorthese mit einem Vibrationselement.
Figur 3 zeigt eine Schale zum Umgreifen eines Unterschenkels für eine Unterschenkelorthese mit einem Vibrationselement.

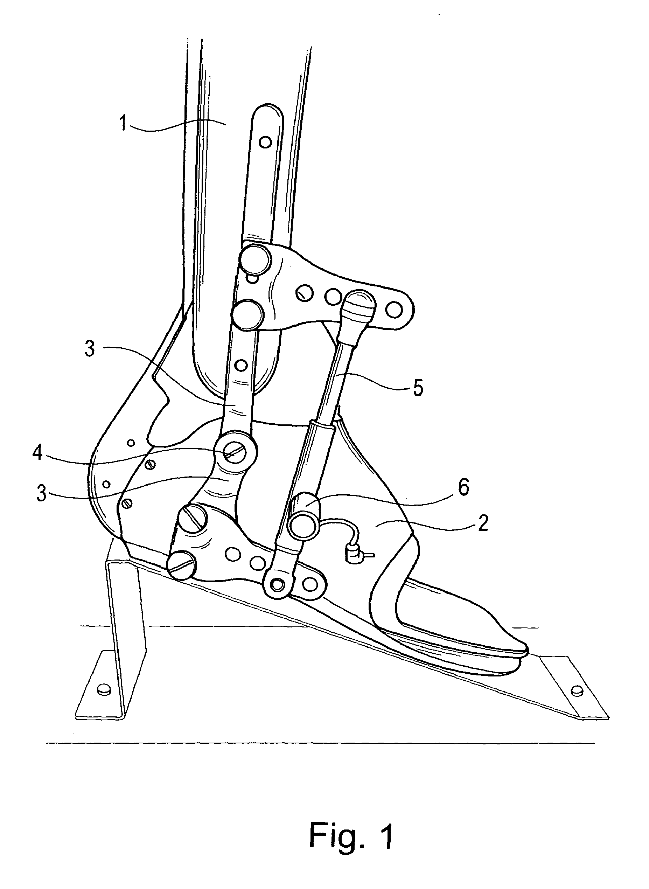

Figur 1 zeigt einen unteren Teil einer Unterschenkelorthese von der Seite. Man erkennt eine Schale 1 zur Anlage an den Unterschenkel und ein Fußteil 2. Das Fußteil 2 ist dazu ausgelegt, einen Fuß im Bereich der Mittelfußknochen vollständig zu umschließen. Es handelt sich hier also um eine ringförmige Fußfassung im Sinne der Erfindung. Unterschenkelschale 1 und Fußteil 2 sind hier über Träger 3 und ein Gelenk 4 aneinander befestigt. Das Gelenk 4 erlaubt ein Anheben bzw. Absenken des Vorderfußes. Die Bewegung um das Gelenk 4 wird durch ein Federelement 5 mit Dämpfungseigenschaften gedämpft, welches parallel zu dem Gelenk 4 und parallel zu diesem ebenfalls an den Trägern 3 befestigt ist. An dem Federelement 5 ist ein Vibrationselement 6 befestigt, welches mit seinen Vibrationen über das Federelement 5 und die Träger 3 auf die Unterschenkelschale 1 und das Fußteil 2 einwirken kann.

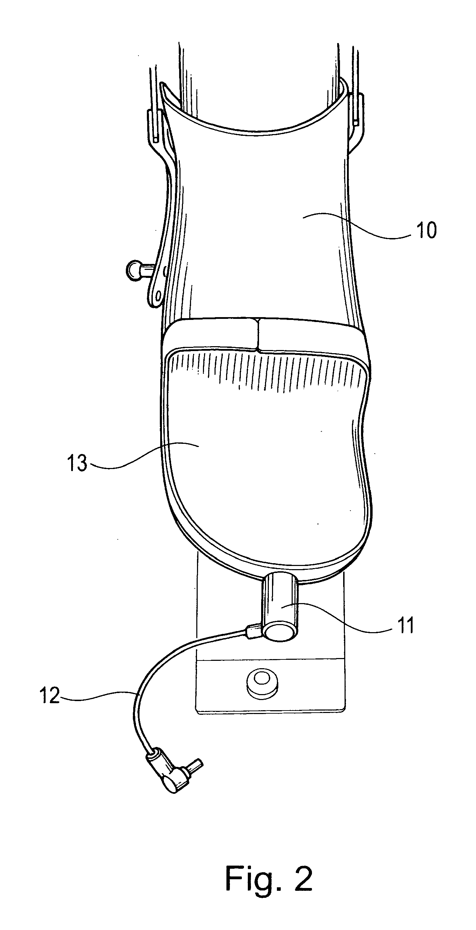

Figur 2 zeigt eine ringförmige Fußfassung 10 mit einem Vibrationselement 11. Das Vibrationselement 11 ist mittig vorne an der Sohle der ringförmigen Fußfassung 10 befestigt. An dem Vibrationselement 11 ist eine Leistungszuführung 12 befestigt. Zur Verbesserung des Tragekomforts ist die ringförmige Fußfassung 10 von Innen mit einer weichen Innenhülle 13 ("Liner") ausgestattet. Bei einer alternativen Ausführungsform ist das Vibrationselement 11 mittig außen unterhalb der Sohle der ringförmigen Fußfassung 10 untergebracht (nicht gezeigt).

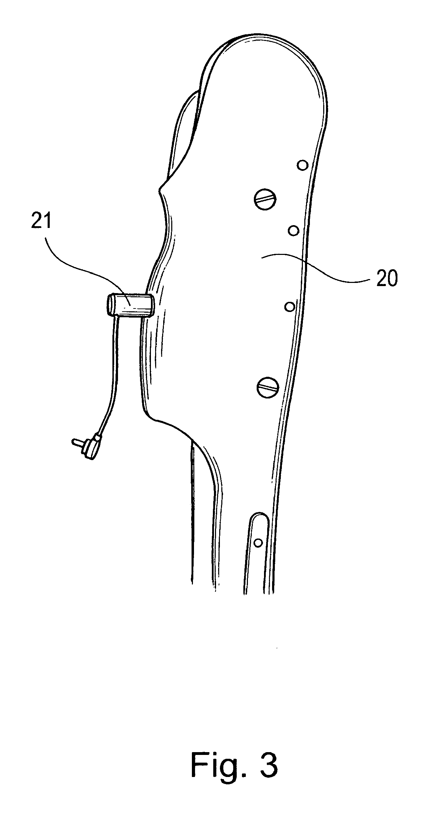

Figur 3 zeigt eine Schale 20 einer Unterschenkelorthese. Ein Vibrationselement 21 ist so an der Schale 20 befestigt, dass es bei getragener Orthese oberhalb der Wadenmuskulatur liegt.

Die in den vorangehenden Figuren gezeigten Vibrationselemente 6, 11 und 21 verfügen jeweils über die Möglichkeit, bezüglich ihrer Schwingungsamplitude und Frequenz eingestellt zu werden. Dazu weisen sie jeweils ein entsprechendes Bedienelement (nicht gezeigt) auf. Alternativ und/oder ergänzend kann der Zeitverlauf der Schwingung und der Amplitude auch programmiert werden und die entsprechenden Steuerungsdaten drahtlos von einem Laptop auf die Vibrationselemente 6, 11 und 21 übertragen werden.

Eine weitere beispielhafte Unterschenkelorthese (nicht gezeigt) weist zwei Vibrationselemente auf, die so zusammenwirken können, dass sich eine gewünschte Vibrationsverteilung entlang der Unterschenkelorthese ergibt.

Bei noch einer weiteren erfindungsgemäßen Unterschenkelorthese (ebenfalls nicht gezeigt) ist eine ringförmige Fußfassung über einen Gummipuffer an eine Unterschenkelschale gekoppelt. Die ringförmige Fußfassung weist in diesem Beispiel ein Vibrationselement auf, die obere Schale jedoch nicht. Entsprechend wirken sich hier die Vibrationen vor allem auf den Bereich der ringförmigen Fußfassung aus.

## Patentansprüche

1. Orthese mit
mindestens einem Vibrationselement (6, 11, 21) zur Erzeugung von Vibrationen in zumindest einem Abschnitt der Orthese,
wobei dieser Abschnitt dazu eingerichtet ist, im an einen Körperteil angelegten Zustand der Orthese auf Kontakt anzuliegen.

2. Orthese nach Anspruch 1, mit einer Fixiereinrichtung (2, 10) zur Sicherung des Kontakts des Abschnitts.

3. Orthese nach Anspruch 2, bei der die Fixiereinrichtung (2, 10) eine zumindest abschnittweise ringförmige Fassung (2, 10) zum zumindest teilweisen Umgreifen einer Extremität aufweist.

4. Orthese nach Anspruch 3, bei der die Fixiereinrichtung (2, 10) eine geschlossene, ringförmige Fassung (2, 10) ist.

5. Orthese nach einem der Ansprüche 3 oder 4, bei der die Fixiereinrichtung (2, 10) eine Fußfassung zum Umgreifen eines Fußes ist.

6. Orthese nach einem der vorangehenden Ansprüche, bei der die Schwingungsamplitude des Vibrationselements (6, 11, 21) vorgebbar ist.

7. Orthese nach einem der vorangehenden Ansprüche, bei der die Schwingungsfrequenz des Vibrationselements (6, 11, 21) vorgebbar ist.

8. Orthese nach einem der vorangehenden Ansprüche, mit zumindest zwei räumlich voneinander getrennten Vibrationselementen (6, 11, 21).

9. Orthese nach Anspruch 2, auch in Kombination mit einem weiteren der vorangehenden Ansprüche, mit einer Schale (1, 20) zur Anlage an einen Unterschenkel und einem die Fixiereinrichtung (2, 10) aufweisenden Fußteil (2, 10).

10. Orthese nach Anspruch 9, bei der ein Vibrationselement (6, 11, 20) an der Sohle des Fußteils (2, 10) angebracht ist.

11. Orthese nach einem der vorangehenden Ansprüche mit einer Schwingungsentkopplung zwischen Teilen der Orthese, welche eine Übertragung von Vibrationen von einem Teil der Orthese auf einen anderen Teil unterdrückt.
